# EUROPÄISCHE PATENTSCHRIFT

(11) **EP 2 398 585 B1**
(45) Veröffentlichungstag und Bekanntmachung des Hinweises auf die Patenterteilung: **14.11.2012**
(21) Anmeldenummer: 10703485.2
(22) Anmeldetag: 16.02.2010
(51) Int. Cl.: B01J 8/06, B01J 19/24, C07C 67/055, C07C 69/15

(54) **VERFAHREN ZUR HERSTELLUNG VON VINYLACETAT**
METHOD FOR PRODUCING VINYL ACETATE
PROCÉDÉ DE PRODUCTION D'ACÉTATE DE VINYLE

(30) Priorität: 19.02.2009 DE 102009001021
(43) Veröffentlichungstag der Anmeldung: 28.12.2011
(73) Patentinhaber: Wacker Chemie AG, 81737 München (DE)
(72) Erfinder: DAFINGER, Willibald, 94133 Röhrnbach (DE); HOLL, Peter, 84547 Emmerting (DE); RUDOLF, Günther, 84453 Mühldorf (DE); SCHÖNLEBEN, Martin, 84367 Reut (DE)
(74) Vertreter: Schuderer, Michael
(86) Internationale Anmeldenummer: PCT/EP2010/051878
(87) Internationale Veröffentlichungsnummer: WO 2010/094660

(56) Entgegenhaltungen:
- WO-A1-2006/042659
- WO-A2-00/54877
- DE-A1- 4 431 949
- DE-A1- 19 914 066
- DE-A1-102007 025 869

## Beschreibung

Die Erfindung betrifft ein Verfahren zur Herstellung von Vinylacetat in einem heterogen katalysierten, kontinuierlichen Gasphasenprozess durch Umsetzung von Ethylen mit Essigsäure und Sauerstoff in einem Rohrbündelreaktor.

Vinylacetat (VAM) wird in einem kontinuierlichen Verfahren unter Rückführung des aufgereinigten Produktstromes hergestellt (Kreisgas-System). Dabei reagiert in einem heterogen katalysierten Gasphasenprozess Ethylen mit Essigsäure und Sauerstoff an Festbett-Katalysatoren, welche im allgemeinen Palladium- und Alkalimetallsalze auf einem Trägermaterial enthalten und zusätzlich noch mit Gold, Rhodium oder Cadmium dotiert sein können.

Die Edukte Ethylen, Sauerstoff und Essigsäure werden in einer exothermen Reaktion (VAM: Δ_{B}H°₂₉₉ = - 176 kJ/mol), im Allgemeinen bei einem Druck von 1 bis 30 bar und einer Temperatur von 130°C bis 200°C, in einem Festbettröhren-Reaktor zu Vinylacetat umgesetzt: C₂H₄ + CH₃COOH + ½ O₂ -> CH₃COOCH=CH₂ + H₂O

Hauptnebenreaktion ist die Ethylen-Totaloxidation zu CO₂:

C₂H₄ + 3 O₂ -> 2 CO₂ + 2 H₂O

Die Bildungswärme beträgt hier Δl_{B}Hº ₂₉₉ = - 1324 kJ/mol ! Der Ethylenumsatz liegt bei etwa 10 %, der Essigsäureumsatz bei 20 bis 30 % und der Sauerstoffumsatz bis zu 90 %.

Bei der Herstellung von Vinylacetat wird ein überwiegend aus Ethylen, Kohlendioxid, Ethan, Stickstoff und Sauerstoff bestehendes Gasgemisch im Kreis geführt. Der Gasstrom wird vor dem Festbettröhrenreaktor mit den Reaktanden Essigsäure, Ethylen und Sauerstoff versetzt und mit Heizdampf betriebenen Wärmetauschern auf Reaktionstemperatur gebracht. Die Anreicherung des Kreisgases mit Essigsäure erfolgt üblicherweise mittels einem mit Heizdampf geheizten Essigsäuresättiger oder Essigsäureverdampfer.

Nach der Reaktion werden die Reaktionsprodukte und nicht umgesetzte Essigsäure aus dem Kreisgas auskondensiert und der Aufarbeitung zugeführt. Nicht auskondensiertes Produkt wird in einem mit Essigsäure betriebenen Wäscher ausgewaschen. Das Kreisgas oder eine Teilmenge davon wird, bevor es erneut mit den Edukten versetzt wird, vom gebildeten Kohlendioxid gereinigt. Die auskondensierten Produkte Vinylacetat und Wasser sowie nicht umgesetzte Essigsäure werden in einem mehrstufigen, üblicherweise mit Heizdampf betriebenem, Destillationsprozess voneinander getrennt. Die üblichen Destillationsschritte sind Entwässerung (gegebenenfalls auch Vorentwässerung), Azeotropdestillation, Rein-VAM-Kolonne, Rückstandsaufarbeitung, sowie Leichtsieder- und Hochsieder-Abtrennung.

Die Reaktionstemperatur im Festbett-Rohrbündelreaktor, im Allgemeinen von 130°C bis 200°C, wird mittels Siedewasserkühlung bei einem Druck von 1 bis 10 bar eingestellt. Dabei wird Wasserdampf, der sogenannte Eigendampf, im Allgemeinen mit einer Temperatur von 120°C bis 185°C, bei einem Druck von 1 bis 10 bar, vorzugsweise 2,5 bis 5 bar, gebildet. Die über die Betriebszeit eines Katalysators nachlassende Aktivität wird durch Erhöhen der Reaktionstemperatur, das heißt des Betriebsdruckes der Siedewasserkühlung, ausgeglichen. Es gilt dabei, zur Schonung des Katalysators, zwecks Erreichung einer möglichst langen Laufzeit, sowie zum Optimieren der Ethylen-Selektivität, durch Minimierung der CO₂-Bildung die Vinylacetat-Reaktion möglichst lange mit einer möglichst niedrigen Reaktionstemperatur zu betreiben.

Im Allgemeinen sind die Festbett-Rohrbündelreaktoren aus mehreren tausend, üblicherweise 2000 bis 6000 dicht gepackten, und vertikal angeordneten, zylinderförmigen Rohren aufgebaut. Für den großtechnischen Einsatz werden dazu Rohre mit einer Länge von 5 bis 6 m und einem inneren Durchmesser von 33 mm bis 40 mm eingesetzt. Das Mantel/Volumen-Verhältnis beträgt im Allgemeinen 100 bis 120 m⁻¹.

Ein Problem bei der Vinylacetat-Herstellung in einem heterogen katalysierten Gasphasenprozess, insbesondere bei zunehmend leistungsfähigeren Katalysatoren, stellt die freigesetzte Reaktionswärme dar. Das Verfahren ist nämlich durch eine deutliche Exothermie gekennzeichnet, weniger bei der Hauptreaktion, als bei der Nebenreaktion der Ethylenoxidation. Selbst bei guten Ethylen-Selektivitätswerten (> 92 %) stammt bei einem Molverhältnis von VAM : CO₂ von ca. 7 : 1, aufgrund der hohen Bildungswärme des CO₂, die Hälfte der Reaktionswärme aus dieser Nebenreaktion! Selbst bei hinsichtlich der Raum-Zeit-Ausbeute (STY) ständig verbesserten VAM-Katalysatoren, kann daher nie gleichzeitig auch die Ethylenselektivät S_{E} so verbessert werden, dass sich als Resultat der STY-Steigerung auch die gesamte Wärmetönung reduzieren würde.

Im Stand der Technik wurde bisher so vorgegangen, dass mit leistungsstärkeren Kreisgasverdichtern höher verdichtet wurde, damit die Strömungsgeschwindigkeit des Kreisgas erhöht wurde, und die axiale Wärmeabführung optimiert wurde. Nachteilig sind dabei die höheren Differenzdrucke in allen Kreisgasapparaturen sowie die schwerer zu kontrollierende, hohe Gasgeschwindigkeit des Kreisgases. Für die herkömmlichen VAM-Katalysatoren, deren STY zwischen 400 bis 700 g VAM /1 Katalysator x h liegt, waren solche Maßnahmen, mit allen genannten Nachteilen ausreichend.

Bei Hochleistungskatalysatoren zur VAM-Herstellung, deren STY deutlich über 700 g VAM /1 Katalysator x h liegt, ist die gebildete Reaktionswärme deutlich höher, mit der Folge, dass die Wärmeabführung in das Kühlmedium Dampf/Wasser, selbst mit den obengenannten Maßnahmen zur Erhöhung der Kreisgasgeschwindigkeit, zu langsam abläuft. In der Folge kommt es im Reaktor nicht nur vermehrt zu lokalen Temperaturerhöhungen (hot spots) und damit einhergehender Ethylen-Selektivitätsminderung, sondern auch zu verkürzten Katalysatorlaufzeiten und zu insgesamt höherer Nebenproduktbildung.

Aus der EP 1033167 A2 ist ein Rohrbündelreaktor mit gestuftem Innendurchmesser bekannt, wobei die Rohre im Bereich des Eintritts des Reaktionsgemisches einen geringeren Durchmesser haben, als im Bereich des Austritts des Reaktionsgemisches. Von einer generellen Verringerung des Durchmessers der Rohre eines Rohrbündelreaktors wird abgeraten, da sich damit die Fertigungskosten des Reaktors (mehr Rohre) erhöhen würden und der Aufwand für dessen Befüllung steigen würde.

Die DE 199 14 066 A1 offenbart ein Verfahren zur Herstellung von Vinylacetat (VAM) in einem Einzelreaktor und nicht in einem Rohrbündelreaktor.

Die WO 2006/042659 A1 betrifft keinen mit Katalysator befüllten Rohrbündelreaktor, sondern offenbart ein Verfahren zur Herstellung von Vinylacetat(VAM) in einem Wandreaktor, wobei die Wände mit Katalysator beschichtet sind.

Der Erfindung lag die Aufgabe zugrunde, das Verfahren zur Herstellung von Vinylacetat durch Umsetzung von Ethylen mit Essigsäure und Sauerstoff in einem heterogen katalysierten, kontinuierlichen Gasphasenprozess dahingehend zu verbessern, dass Hochleistungskatalysatoren mit einer Raumzeitausbeute (STY) von mehr als 700 g VAM/1 Katalysator x Stunde eingesetzt werden können, unter Vermeidung von lokalen Temperaturerhöhungen (hot spots) und damit einhergehender Ethylen-Selektivitätsminderung.

Gegenstand der Erfindung ist ein Verfahren zur Herstellung von Vinylacetat (VAM) durch Umsetzung von Ethylen mit Essigsäure und Sauerstoff in einem Festbett-Rohrbündelreaktor in einem heterogen katalysierten, kontinuierlichen Gasphasenprozess, dadurch gekennzeichnet, dass zur Katalyse ein Hochleistungskatalysator mit einer Raum-Zeit-Ausbeute von > 700 g VAM/1 Katalysator x Stunde eingesetzt wird, und dass der Festbett-Rohrbündelreaktor Rohre umfasst mit einem Verhältnis von Mantelinnenfläche zu Volumen von ≥ 130 m⁻¹.

Die zur Herstellung von Vinylacetat eingesetzten Festbett-Rohrbündelreaktoren bestehen in der Regel aus einem zylinderförmigen Behälter, in welchem eine Vielzahl von zylindrischen Rohren (das Rohrbündel) vertikal angeordnet ist. Die Rohre sind im Bündel dicht gepackt angeordnet. Üblicherweise umfasst ein Festbett-Rohrbündelreaktor für den großtechnischen Einsatz 2000 bis 6000 Rohre. Im Allgemeinen beträgt der Abstand zwischen den Rohren, das heißt zwischen den zentrischen Innenachsen der Rohre, 30 bis 40 mm. Die Zwischenräume zwischen den Rohren selbst, und den Rohren und dem Behälter, werden zur Kühlung von einem Wasser/Wasserdampf-Gemisch als Wärmetauschmittel durchströmt.

Für den großtechnischen Einsatz gebräuchlich sind dabei Festbett-Rohrbündelreaktoren mit einer Bauhöhe von einigen Metern, das heißt im Allgemeinen einer Rohrlänge von 4 bis 7 m. Bei den Rohren handelt es sich um Stahlrohre mit einer Wanddicke von im Allgemeinen 1 bis 3 mm. Die Rohre sind dabei erfindungsgemäß so dimensioniert, dass deren Verhältnis von Mantelfläche (im Rohrinneren) zu Rohrvolumen ≥ 130 m⁻¹, vorzugsweise 130 bis 160 m⁻¹, beträgt. Der Innendurchmesser der Rohre ist vorzugsweise über deren gesamte Länge konstant.

Es zeigte sich, dass gegenüber dem hergebrachten M:V-Verhältnis von etwa 100 bis 120 m⁻¹, insbesondere bei einem Mantelfläche/Volumen-Verhältnis von 130 bis 160 m⁻¹ Verbesserungen hinsichtlich der Wärmeabführung von > 30 % erreichbar sind. Eine noch deutlichere Wärmeabführung ist zwar mit noch wesentlich geringeren Rohrdurchmessern erreichbar, aber dann beginnt die Rohrbefüllung unwirtschaftlich zu werden, bzw. durch die immer enger werdenen Rohre entstehen bei der Befüllung erhebliche Brücken- und Lückenbildungen der Katalysatorkörper, besonders an den Wandungen. Dem kann zwar begegnet werden durch eine Verkleinerung der Katalysatorteilchen, dies führt aber wiederum zu einer Verringerung des Lückenvolumens und daher zu einem beträchtlichen Differenzdruckanstieg.

Zur Katalyse werden die Rohre in bekannter Weise mit partikelförmigen Hochleistungskatalysatoren mit einer Raum-Zeit-Ausbeute von > 700 g VAM / 1 Katalysator x Stunde, vorzugsweise 800 bis 1500 g VAM / 1 Katalysator x Stunde, besonders bevorzugt 900 bis 1500 g VAM / 1 Katalysator x Stunde, befüllt. Es sind im Allgemeinen Trägerkatalysatoren, basierend auf einem inerten, anorganischen Trägermaterial wie Titanoxiden, Siliciumoxiden, Aluminiumoxiden, welche mit einer Palladiumverbindung in Kombination mit Alkalimetallsalzen beschichtet sind, und zusätzlich noch mit Gold, Rhodium oder Cadmium dotiert sein können. Die Trägerkatalysatoren können in Form von Kugeln, Zylindern oder Ringen vorliegen, wobei deren Abmessungen den eingesetzten Rohren angepasst sind, und im Allgemeinen Längen bzw. Breiten von 3 bis 10 mm bzw. Durchmesser von 3 bis 6 mm aufweisen.

Die auffälligste Verbesserung bei der erfindungsgemäßen Vorgehensweise war die deutliche Erhöhung der Ethylen-Selektivität, und die parallel dazu deutlich ansteigende Raum-Zeit-Ausbeute (STY). Auch die Sauerstoff-Selektivität verbesserte sich auffällig. Außerdem zeigen die Werte in den Tabellen, dass eine um fast 10 % geringere Menge des Kondensatumlaufes angewandt werden konnte. Somit ist über den Selektivitäts- und STY-Gewinn hinaus auch eine Stromeinsparung an den Kondensat-Umlaufpumpen zu erreichen.

### Beispiel:

Es wurde der thermische Reaktionsverlauf in zwei Rohr-Reaktoren, ausgerüstet mit Rohren mit einem Durchmesser von 33 mm (Vergleich) bzw. Rohren mit einem Durchmesser von 27 mm (erfindungsgemäß), verglichen.
A) Ein Reaktor mit vier Rohren (6,30 m lang, innerer Durchmesser 33 mm; M/V = 120 m⁻¹) ; Kühlmedium Wasser/Dampf mit einem Kondensatumlauf von 1300 kg/h; externe Wärmeabfuhr über eine Dampftrommel. Ein Rohr ausgestattet mit einer Temperaturmesskette, alle 20 cm ist eine Messstelle angebracht.
B) Ein Reaktor mit 7 Rohren (6,30 m lang, innerer Durchmesser 27 mm; M/V = 148 m⁻¹), Kühlmedium Wasser/Dampf mit einem Kondensatumlauf von 1240 kg/h; externe Wärmeabfuhr über eine Dampftrommel. Zwei Rohre ausgestattet mit einer Temperaturmesskette, alle 20 cm ist eine Messstelle angebracht.

Beide Reaktoren wurden befüllt mit einem VAM-Katalysator KL 7900 SH von KataLeuna/Deutschland (Pd 1,5 Gew.-%; Au 1 Gew.-%) Da sich die Ergebnisse bei Verwendung der Raumzeitausbeute (STY = g VAM/1 Katalysator x Stunde) auf einen 1-Liter Katalysator beziehen, spielt die größere Katalysatormenge im 7-RohrReaktor keine Rolle.

In einer VAM-Pilotanlage mit den beiden Reaktoren A und B wurde ein identischer Katalysator bei identischen Reaktionsbedingungen getestet.

Die Reaktionsbedingungen, Raumzeit-Ausbeute und Selektivität sind in Tabelle 1 (nach 300 h), Tabelle 2 (nach 400 h), Tabelle 3 (nach 1000 h) und Tabelle 4 (nach 1500 h) dargestellt. Der Temperaturverlauf in den Reaktoren ist in Abbildung 1 (nach 300 h), Abbildung 2 (nach 400 h), Abbildung 3 (nach 1000 h) und Abbildung 4 (nach 1500 h) dargestellt.

Bereits nach 300 Stunden zeigte sich bei der erfindungsgemäßen Vorgehensweise ein signifikant verbesserter Temperaturverlauf. Der Temperaturunterschied zwischen dem Vergleichsreaktor und dem erfindungsgemäß ausgestatteten Reaktor betrug bis zu mehr als 10°C, welcher nach einer Betriebszeit von 400 h noch deutlicher wurde. Es konnte damit eine Steigerung der Ethylen-Selektivität von mehr als 1 % in der Anfangszeit (300 bis 400 h) erhalten werden. Noch deutlicher wurden die Vorteile mit zunehmender Versuchsdauer, trotz nochmals gesteigerter Kondensatumlaufmenge beim großrohrigen Reaktor, aber gleichbleibendem Umlauf bei den 27 mm Rohren, das heißt geringerer Kühlung beim 27 mm Rohrreaktor, verbesserte sich bei Letzterem die Ethylenselektivität um 2 %.

**Tabelle 1: (Bedingungen und Performance nach 300 Stunden)**

| **Rohrdurchmesser** | **33 mm** | **27 mm** |
|---|---|---|
| Kreisgasdruck (pₑ) | 8, 5 | 8,5 |
| AcOH (%) | 12,0 | 11,5 |
| C₂H₄ (%) | 65 | 65 |
| Temperatur (°C) | 150 | 150 |
| O₂ (%) | 6,8 | 6,8 |
| Kondensatumlauf (kg/h) | 1340 | 1240 |
| GHSV (1/h) | 3740 | 3720 |
| (GHSV = Gas Hourly Space Velocity) | | |
| STY (kg VAM/1 Kat x h) | 850 | 850 |
| Selektivität C₂H₄ | 92,1 | 93,3 |
| Selektivität O₂ | 65 - 70 | 75 - 80 |

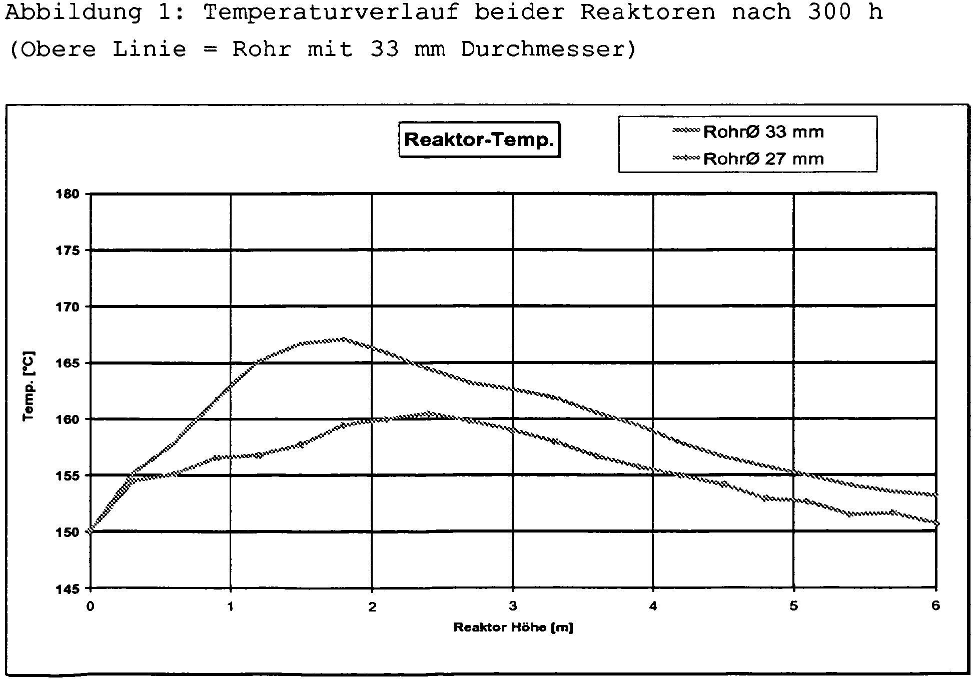

**Tabelle 2: (Bedingungen und Performance nach 400 Stunden)**

| **Rohrdurchmesser** | **33 mm** | **27 mm** |
|---|---|---|
| Kreisgasdruck (pₑ) | 8,5 | 8,5 |
| AcOH (%) | 12,3 | 11,5 |
| C₂H₄ (%) | 65 | 65 |
| Temperatur (°C) | 150 | 150 |
| O₂ (%) | 8,6 | 8,8 |
| Kondensatumlauf (kg/h) | 1340 | 1240 |
| GHSV (1/h) | 3750 | 3750 |
| STY (kg VAM/1 Kat x h) | 900 | 935 |
| Selektivität C₂H₄ | 91,0 | 92,3 |
| Selektivität O₂ | 65 - 70 | 75 - 80 |

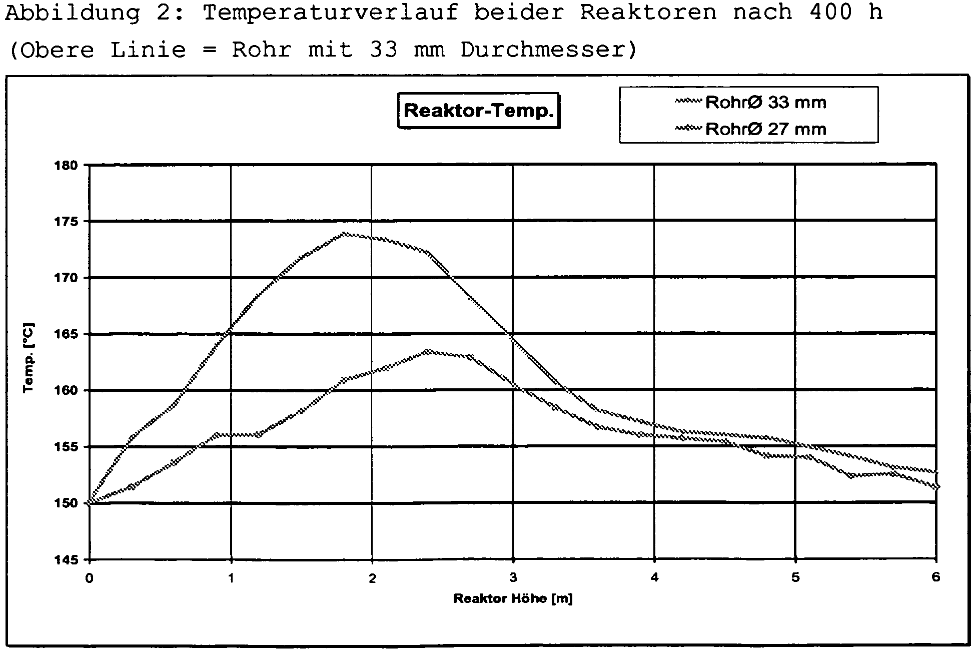

**Tabelle 3: (Bedingungen und Performance nach 1000 Stunden)**

| **Rohrdurchmesser** | **33 mm** | **27 mm** |
|---|---|---|
| C₂H₄ (%) | 61,3 | 63,1 |
| Temperatur (°C) | 150 | 155 |
| Kondensatumlauf (kg/h) | 1530 | 1230 |
| GHSV (1/h) | 3660 | 3820 |
| STY (kg VAM/1 Kat x h) | 920 | 980 |
| Selektivität C₂H₄ | 90,9 | 93,3 |
| Selektivität O₂ | 55 - 60 | 65 - 70 |

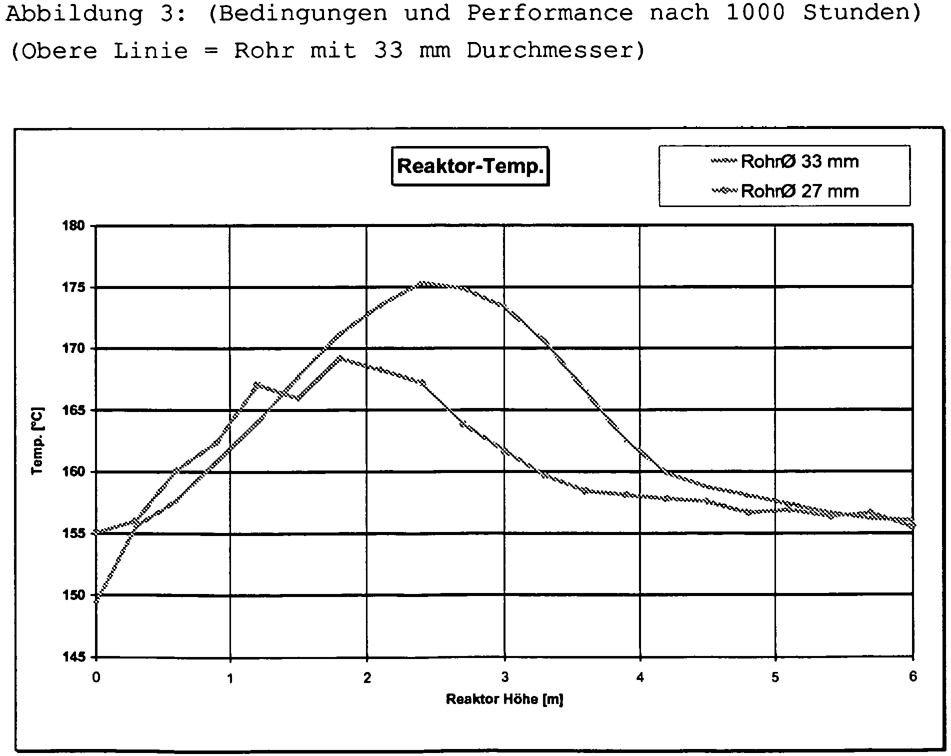

**Tabelle 4: (Bedingungen und Performance nach 1500 Stunden)**

| **Rohrdurchmesser** | **33 mm** | **27 mm** |
|---|---|---|
| C₂H₄ (%) | 61,0 | 62,0 |
| Temperatur (°C) | 150 | 155 |
| Kondensatumlauf (kg/h) | 1750 | 1260 |
| GHSV (1/h) | 3600 | 3800 |
| STY (kg VAM/1 Kat x h) | 930 | 980 |
| Selektivität C₂H₄ | 91,4 | 93,4 |
| Selektivität O₂ | 60 - 65 | 70 - 75 |

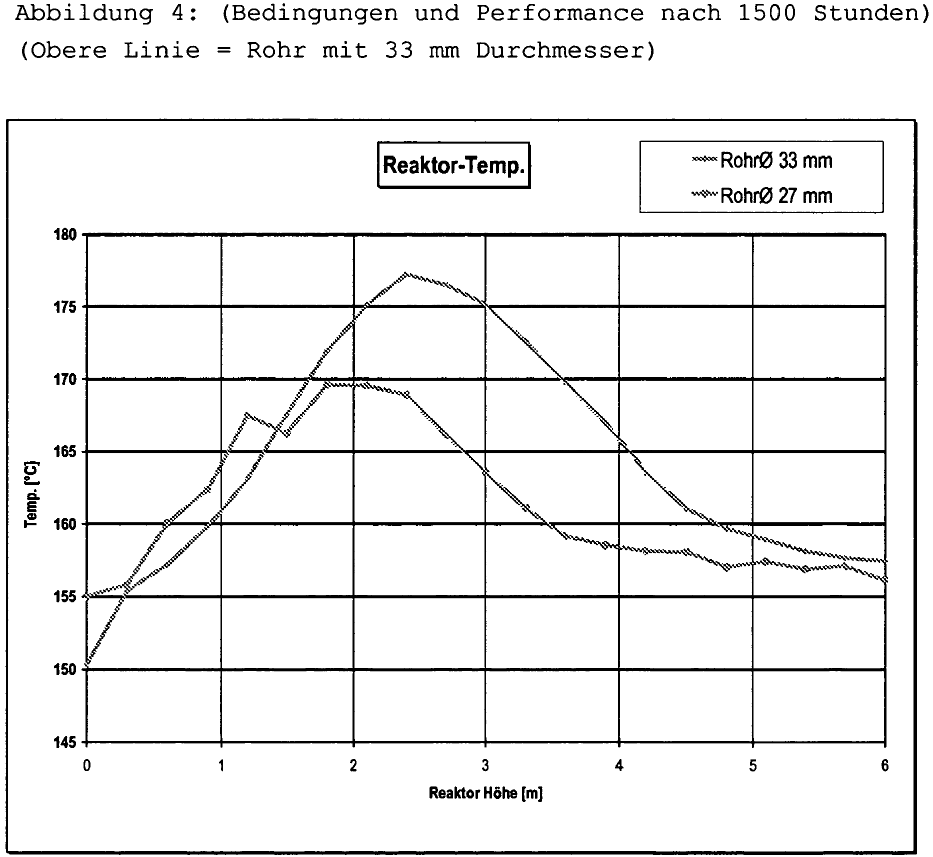

## Patentansprüche

1. Verfahren zur Herstellung von Vinylacetat (VAM) durch Umsetzung von Ethylen mit Essigsäure und Sauerstoff in einem Rohrbündelreaktor in einem heterogen katalysierten, kontinuierlichen Gasphasenprozess, **dadurch gekennzeichnet, dass** zur Katalyse ein Hochleistungskatalysator mit einer Raum-Zeit-Ausbeute von über 700 g VAM/1 Katalysator x Stunde eingesetzt wird, und dass der Rohrbündelreaktor Rohre umfasst mit einem Verhältnis von Mantelinnenfläche zu Volumen von ≥ 130 m⁻¹.

2. Verfahren gemäß Anspruch 1 **dadurch gekennzeichnet, dass** das Verhältnis von Mantelinnenfläche zu Volumen von 130 bis 160 m⁻¹ beträgt.

3. Verfahren nach Anspruch 1 oder 2, **dadurch gekennzeichnet, dass** der Rohrbündelreaktor Rohre mit einer Rohrlänge von 4 Meter bis 7 Meter umfasst.

4. Verfahren nach Anspruch 1 bis 3, **dadurch gekennzeichnet, dass** die Zwischenräume im Rohrbündelreaktor zur Kühlung von einem Wasser/Wasserdampf-Gemisch als Wärmetauschmittel durchströmt wird.

## Claims

1. Process for preparing vinyl acetate monomer (VAM) by reacting ethylene with acetic acid and oxygen in a tube bundle reactor in a heterogeneously catalysed, continuous gas phase process, **characterized in that** a high-performance catalyst with a space-time yield of more than 700 g of VAM/1 of catalyst x hour is used for catalysis, and **in that** the tube bundle reactor comprises tubes with a ratio of inner surface area to volume of ≥ 130 m⁻¹.

2. Process according to Claim 1, **characterized in that** the ratio of inner surface area to volume is from 130 to 160 m⁻¹.

3. Process according to Claim 1 or 2, **characterized in that** the tube bundle reactor comprises tubes with a tube length of 4 metres to 7 metres.

4. Process according to Claims 1 to 3, **characterized in that** a water/steam mixture as a heat exchange medium flows through the interstices in the tube bundle reactor for cooling.

## Revendications

1. Procédé pour la préparation d'acétate de vinyle (VAM) par transformation d'éthylène avec de l'acide acétique et de l'oxygène dans un réacteur à faisceau tubulaire dans un procédé continu en phase gazeuse, catalysé par voie hétérogène, **caractérisé en ce qu'**un catalyseur à haute performance présentant un rendement espace-temps supérieur à 700 g de VAM/1 de catalyseur x heure est utilisé pour la catalyse et **en ce que** le réacteur à faisceau tubulaire comprend des tubes présentant un rapport de la surface interne de l'enveloppe au volume ≥ 130 m⁻¹.

2. Procédé selon la revendication 1, **caractérisé en ce que** le rapport de la surface interne de l'enveloppe au volume est de 130 à 160 m⁻¹.

3. Procédé selon la revendication 1 ou 2, **caractérisé en ce que** le réacteur à faisceau tubulaire comprend des tubes présentant une longueur de tube de 4 mètres à 7 mètres.

4. Procédé selon les revendications 1 à 3, **caractérisé en ce que** les espaces intermédiaires dans le réacteur à faisceau tubulaire sont traversés par un mélange eau/vapeur d'eau comme agent d'échange thermique en vue du refroidissement.
